# EUROPEAN PATENT SPECIFICATION

(11) **EP 2 226 045 B1**
(45) Date of publication and mention of the grant of the patent: **07.08.2013**
(21) Application number: 08792501.2
(22) Date of filing: 15.08.2008
(51) Int. Cl.: A61F 13/49, A61F 13/496, A61F 13/511, A61F 13/15

(54) **DISPOSABLE PANTS-TYPE WEARING ARTICLE**
TRAGBARER ARTIKEL VOM EINWEGHÖSCHENTYP
ARTICLE D'HABILLEMENT JETABLE DE TYPE CULOTTE

(30) Priority: 28.12.2007 JP 2007340908
(43) Date of publication of application: 08.09.2010
(73) Proprietor: Uni-charm Corporation, Ehime 799-0111 (JP)
(72) Inventor: OTSUBO, Toshifumi, Kanonji-shi Kagawa 769-1602 (JP)
(74) Representative: Eke, Philippa Dianne
(86) International application number: PCT/JP2008/064655
(87) International publication number: WO 2009/084266

(56) References cited:
- GB-A- 2 287 393
- JP-A- H09 510 384
- JP-A- 2002 501 417
- JP-A- 2007 296 314
- US-A1- 2007 239 129

## Description

### TECHNICAL FIELD

The present invention relates to disposable pants-type wearing articles suitable for use as disposable diapers or training pants.

### RELATED ART

It is conventional to provide wearing articles, such as a disposable diaper with means serving to prevent bodily fluids from leaking out beyond the side edges of crotch region of a wearing article.

For example, US Patent Publication No. 4,695,278 (PATENT DOCUMENT 1) discloses an open-type diaper wherein a crotch region is provided with dual cuffs each extending along the associated side edge of the crotch region in the longitudinal direction of the diaper in an elastically stretchable and contractible manner. When such a diaper is put on the wearer's body, the dual cuffs elastically fit around the wearer's legs and thereby prevent bodily fluids from leaking out beyond the respective side edges of the crotch region.

The absorbent article disclosed in Japanese Laid-Open Patent Application Gazette No. 1996-56985 (PATENT DOCUMENT 2) is of pants-type provided along the opposite side edges of the crotch region with pieces of sheet referred to as the side members adapted to close at least partially the respective leg-openings.
PATENT DOCUMENT 1: US Patent Publication No. 4,695,278
PATENT DOCUMENT 2: Japanese Laid-Open Patent Application Gazette No. 1996-56985

US 2007/0239129 discloses a disposable pull-on type diaper with a separator interposed between and inner surface of an absorbent chassis and the wearer's skin. The front and rear ends of the separator are permanently bonded in front and rear joint zones by hot melt adhesives. GB 2287393 discloses an absorbent article having longitudinally extending flaps connected to an inner casing sheet, which flaps are joined together in the crotch part of the article.

### DISCLOSURE OF THE INVENTION

### PROBLEM TO BE SOLVED BY THE INVENTION

During use of the diaper, there is a possibility that an amount of feces having its viscosity reduced by urine mixed therewith may spread and contaminate the wearer's skin and/or leak out from the diaper. Certainly it will be possible for the diaper disclosed in PATENT DOCUMENT 1 and the absorbent article disclosed in PATENT DOCUMENT 2 to prevent an amount of feces having its vicinity reduced from leaking out from the diaper or the article, but it is impossible to prevent an amount of feces from being mixed with urine.

In view of the problem as has been described above, it is a principal object of the present invention to improve the conventional disposable pants-type wearing article so as to prevent an amount of feces from being mixed with urine in the crotch region.

### MEASURE TO SOLVE THE PROBLEM

According to one aspect of the present invention, there is provided a disposable pants-type wearing article having a front-to-rear direction, a transverse direction and a longitudinal direction being orthogonal one to another, and comprising a crotch region, a front waist region extending forward from the crotch region and a rear waist region extending rearward from the crotch region, the front and rear waist regions cooperating together to define a waist-opening while the front and rear waist regions cooperate with the crotch region to define a pair of leg-openings, a peripheral edge of the waist-opening as well as respective peripheral edges of the leg-openings being elastically stretchable and contractible in circumferential directions of the respective openings, and the wearing article further has a longitudinal center line extending in the longitudinal direction to bisect respective dimensions of the front and rear waist regions as measured in the transverse direction and a transverse center line extending in the transverse direction to bisect a dimension of the waist-opening as measured in the front-to-ear direction.

The present invention is **characterized in that** the crotch region is provided on the inward side of the inner surface of the crotch region with a functional sheet extending across the crotch region and bonded to the inner surface of the crotch region along opposite side edges thereof but left free from the inner surface of the crotch region between the opposite side edges, the functional sheet defines a partition wall bisecting the crotch region in the front-to-rear direction and a pair of leg-cuffs surrounding respective portions of the wearer's legs from inside thereof and thereby protecting the portions from contamination with bodily fluids and, with the crotch region folded back along the transverse center line so that the front and rear waist regions may be put flat together, the functional sheet also is folded back along the transverse center line and in contact, on the transverse center line, with the inner surface of the crotch region, respective halves of the functional sheet folded back being formed in respective upper portions thereof with joint zones on both sides of the longitudinal center line, the respective halves of the functional folded back being joined together in the joint zones, the respective upper portions being bisected between the joint zones formed on the both sides of the longitudinal center line so that the respective upper portions bisected in this manner may define the leg-cuffs, respectively, and a portion of the functional sheet extending below the upper portions may be continuous in the transverse direction so as to define the partition wall.

According to the invention, each of the upper portions of the functional sheet folded back onto itself occupies 1/4 to 3/4 of a dimension of the functional sheet having been folded back onto itself as measured in the longitudinal direction.

According to another preferred embodiment of the invention, the functional sheet is joined to the inner surface of the crotch region on the transverse center line or in the vicinity thereof.

According to still another preferred embodiment of the invention, the leg-cuffs are elastically stretchable and contractible in circumferential direction partially around the wearer's legs so that the leg-cuffs may elastically fit partially around the wearer's legs.

### EFFECT OF THE INVENTION

With the disposable pants-type wearing article put on the wearer's body, the functional sheet extending across the crotch region forms the leg-cuffs adapted to cover the portions of the wearer's legs from inside and thereby to protect the wearer's leg from contamination with body waste. The functional sheet further serves to form the partition wall adapted to divide the crotch region into front and rear halves and thereby to prevent urine discharged onto the front half of the crotch region and feces discharged onto the rear half of the crotch region from being mixed together.

The other effects provided by the preferred embodiments of the invention will be understood from the following description.

### BRIEF DESCRIPTION OF THE DRAWINGS

[FIG. 1] Fig. 1 is partially cutaway perspective view of the pants-type diaper.
[FIG. 2] Fig. 2 is sectional view taken along a line II-II in Fig. 1
[FIG. 3] Fig. 3 is a partially cutaway flatly developed plan view of the pants-type diaper of Fig. 1.
[FIG. 4] Fig. 4 is a view similar to Fig. 3, showing a preferable embodiment.

### IDENTIFICATION OF REFERENCE NUMERALS USED IN THE DRAWINGS

- 1: pants-type diaper
- 6: crotch region
- 7: front waist region
- 8: rear waist region
- 11: waist-opening
- 12: leg-openings
- 13: peripheral edge of leg-opening (periphery, side edge)
- 20: functional sheet
- 20a, 20b: leg-cuffs
- 20c: partition wall
- 23, 23a, 23b: joint zones
- A: front-to-rear direction
- B: transverse direction
- C: longitudinal direction
- F-F: front-to-rear center line
- G-G: transverse center line
- H-H: longitudinal center line

### DESCRIPTION OF THE BEST MODE FOR WORKING OF THE INVENTION

Details of a disposable pants-type wearing article according to the present invention will be more fully understood from the description of pants-type diaper as one of the embodiments thereof.

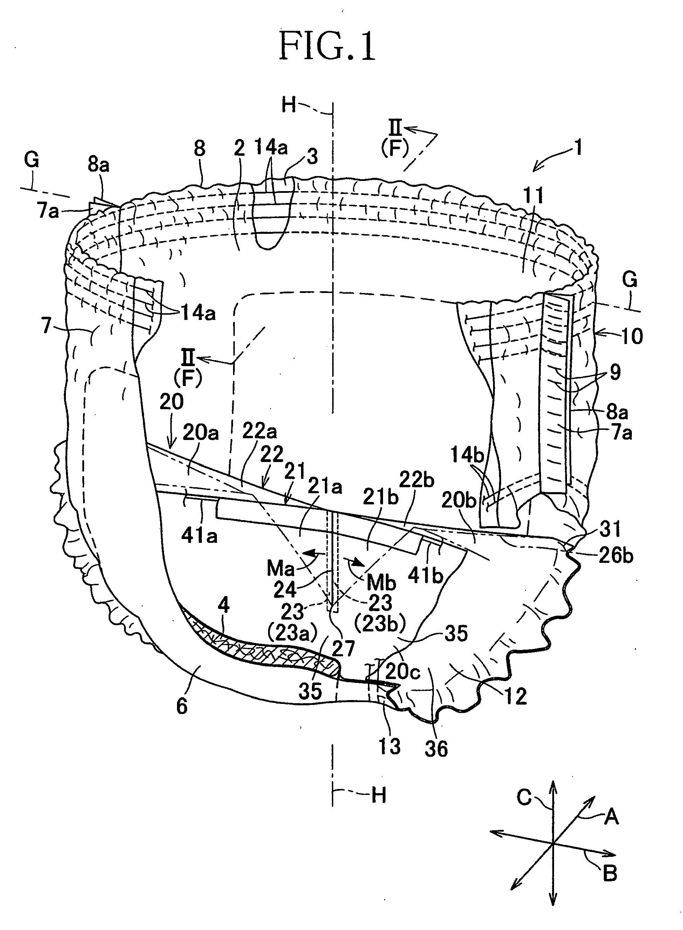

Fig. 1 is a partially cutaway perspective view of a pants-type diaper 1. The pants-type diaper 1 has a pants-shaped chassis 10 comprising a liquid-pervious topsheet 2, a liquid-impervious backsheet 3 and a body fluid absorbent core 4 sandwiched between these two sheets 2, 3 wherein a front-to-rear direction, a transverse direction and a longitudinal direction of the diaper 1 are designated in Fig. 1 by A, B and C, respectively. The chassis 10 has a crotch region 6, a front waist region 7 extending forward from the crotch region 6 and a rear waist region 8 extending rearward from the crotch region 6 wherein opposite side edges 7a, 8a of the front and rear waist regions 7, 8 are put flat and sealed together at a plurality of joint zones 9 arranged intermittently in the longitudinal direction C along the respective side edges 7a, 8a. Thereupon the front and rear waist regions 7, 8 cooperate with each other to define a waist-opening 11 and cooperate also with the crotch region 6 to define a pair of leg-openings 12. Along a peripheral edge of the waist-opening 11, a plurality of waist-surrounding elastic members 14a are sandwiched between the topsheet 2 and the backsheet 3 so as to extend circumferentially and bonded under tension to at least one of the topsheet 2 and the backsheet 3. In a similar manner, each of the leg-openings 12 is provided along its peripheral edge 13 with a plurality of leg-surrounding elastic members 14b sandwiched between the topsheet 2 and the backsheet 3 so as to extend circumferentially and bonded under tension to at least one of the topsheet 2 and the backsheet 3. In this way, leg-surrounding annular elasticized regions are defined along the respective peripheral edges 13 of the leg-openings 12.

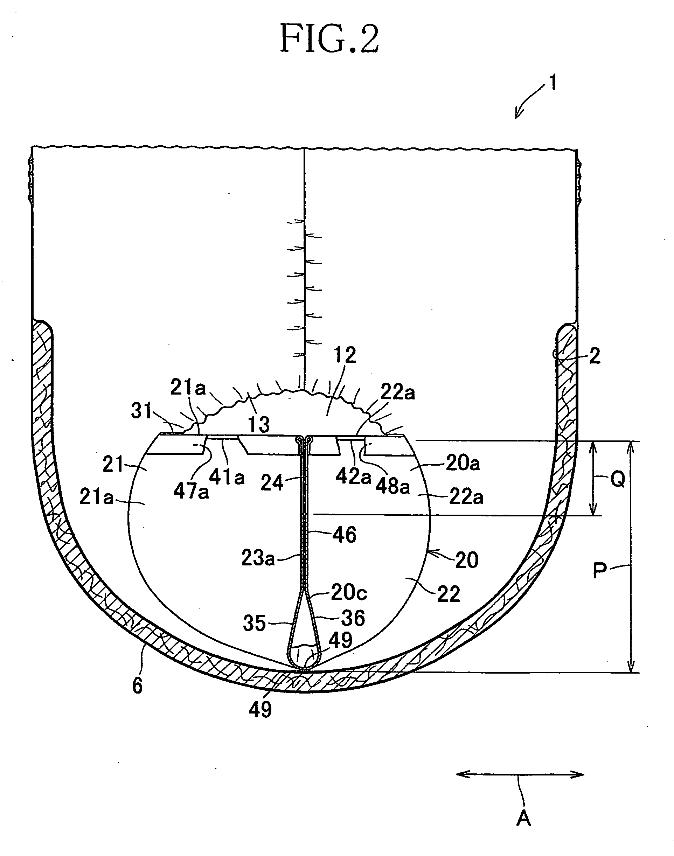

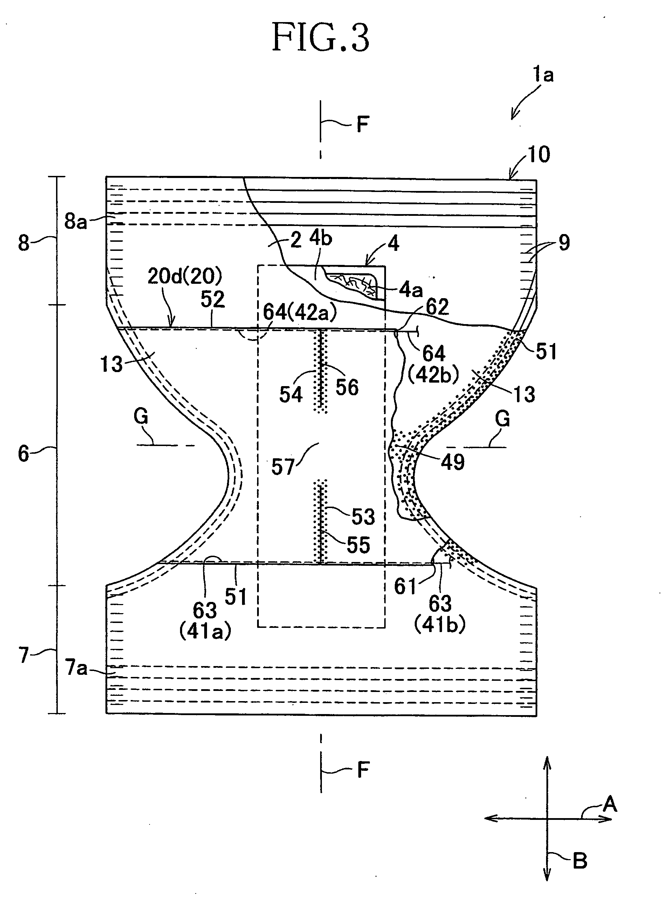

The diaper 1 further includes a functional sheet 20 lying on the inward side of the topsheet 2. This functional sheet 20 is made of soft nonwoven fabric or plastic film, more preferably of soft and liquid-impervious nonwoven fabric, plastic film or the like. The functional sheet 20 made of such material comprises a front half 21 extending forward with respect to a transverse center line G-G extending in the transverse direction B to bisect a diameter of the waist-opening 11 as measured in the front-to-rear direction A and a rear half 22 with respect to the transverse center line G-G. The functional sheet 20 is joined along opposite side edges 31 thereof to the peripheral edges 13 of the respective leg-openings 12 (See Fig. 2). In the intermediate region of the functional sheet 20 defined between the opposite side edges 31 thereof is left free from the topsheet 2. The front half 21 and the rear half 22 of the functional sheet 20 respectively have upper portions thereof joined together at a joint zone 23 (See Figs. 2 and 3) including a first joint zone 23a and a second joint zone 23b defined along and on both sides of a vertical center line H-H extending in the longitudinal direction C so as to bisect a diameter of the waist-opening 11 as measured in the transverse direction B. The respective upper portions of the front half 21 and the rear half 22 are partially separated in the transverse direction B by a slit 24 (See Fig. 2) defined between the first and second joint zones 23a, 23b and extending in the vertical center line H-H. In this way, the upper portion of the front half 21 is divided in first and second upper portions 21a, 21b and the upper portion of the rear half 22 is divided in first and second upper portions 22a, 22b. The first upper portion 21a of the front half 21 and the first upper portion 22a of the rear half 22 are joined together in the first joint zone 23a to form a cuff 20a adapted to partially surround the right leg of the wearer (not shown). In a similar way, the second upper portion 21b of the front half 21 and the second upper portion 22b of the rear half 22 are joined together in the second joint zone 23b to form a cuff 20b adapted to partially surround the left leg of the wearer. The first upper portion 21a of the front half 21 and the first upper portions 22a of the rear half 22 gradually draw apart from each other in a V-shape to ensure good fitness to the right leg and, in a similar way, the second upper portion 21b of the front half 21 and the second upper portion 22b of the rear half 22 gradually draw apart from each other in a V-shape to ensure good fitness to the left leg. The leg-surrounding cuffs 20a, 20b are in an independent relationship with each other because of the presence of the slit 24 lying between these cuffs 20a, 20b so that, with the diaper 1 put on the wearer's body, the respective cuffs 20a, 20b are free to move without any interference with each other. The front half 21 and the rear half 22 of the functional sheet 20 respectively have a lower portion 35 and a lower portion 36 both lying below a lower end 27 of the slit 24 which are contiguous to each other, extending across the crotch region 6 and cooperating with each other to form a partition wall 20c bisecting the crotch region 6 in the front-to-rear direction A. It should be understood here that the first joint zone 23a and the second joint zone 23b constituting the joint zone 23 are contiguous to each other below the lower end 27 of the slit 24. It should be also understood that the vertical line H-H is the line bisecting respective dimensions of the crotch region 6 and the front and rear waist regions 7, 8 as measured in the transverse direction B.

The functional sheet 20 as has been described above is made of sheet material which is not elastically stretchable and provided along respective upper edges of the first and second upper portions 21a, 21b of the front half 21 as well as the rear half 22 with elastic members 41a, 41b, 42a, 42b attached under tension thereto (See Figs. 2 and 3). In this way, the leg-surrounding cuffs 20a, 20b are moved in a direction indicated by an arrow Ma and in a direction indicated by an arrow Mb, respectively, under contraction of these elastic members 41a, 42a, 41b, 42b to positions indicated by imaginary lines. In consequence of such movement, the preferred leg-surrounding cuffs 20a, 20b reliably fit to inner sides of the respective legs and thereby serve as means effective to protect the wearer's legs from being contaminated with body waste such as urine. With the diaper 1 put on the wearer's body, these elastic members 41a, 41b, 42a, 42b extending in the transverse direction as viewed in Figs 1, 2 and 3 are adapted to stretch or contract the leg-surrounding cuffs 20a, 20b in the circumferential direction. The lower portion 35 of the front half 21 and the lower portion 36 of the rear half 22 of the functional sheet 20 extending across the crotch region 6 serve to prevent any amount of urine (not shown) discharged onto the front half 21 of the functional sheet 20 from flowing rearward and to prevent any amount of feces (not shown) discharged onto the rear half 22 from flowing forward. In this manner, the presence of the functional sheet 20 enables to provide the diaper 1 with two advantageous effects at once, i.e., the effect to protect the wearer's legs from contamination with urine and/or feces and the effect to prevent urine and feces from being mixed together.

Fig. 2 is a sectional view taken along a line II-II in Fig. 1 wherein this line II-II conforms not only to the front-to-rear centerline F-F bisecting the diaper 1 in the transverse direction B thereof and extending in the front-to-rear direction A (See Fig. 3 also) but also to the line along which the slit 24 extends. Normally, the crotch region 6 bows in U-shape and the opposite side edges 13 thereof define the leg-openings 12. The functional sheet 20 includes the first upper portion 21a of the front half 21 and the first upper portion 22a of the rear half 22 bonded to each other in the first joint zone 23a containing hot melt adhesive 46. Along respective upper edges of the first upper portion 21a of the front half 21 and the first upper portion 22a of the rear half 22, the functional sheet 20 is folded back so as to form sleeves 47a, 48a within which these sleeves 47a, 48a, the elastic members 41a, 42a attached to the functional sheet 20. Fig. 2 shows these elastic members 41a, 42a as being stretched in the transverse direction B. While the functional sheet 20 has the lower portion 35 of the front half 21 and the lower portion 36 of the rear half 22a spaced from each other in the front-to-rear direction A, the functional sheet 20 is bonded to the topsheet 2 by means of hot melt adhesive 49 on or in the vicinity of the transverse center line G-G (See Fig. 1) defining a border between these portions 35, 36. As will be apparent from Fig. 2, the lower portion 35 of the front half 21 and the lower portion 36 of the rear half 22 cooperate with each other to form the partition wall 20c of dual structure extending across the crotch region 6 to separate the front and rear halves from each other. Referring to Fig. 2, the functional sheet 20 folded in two has a height P is dimensioned so that the functional sheet 20 does not extend beyond respective tops of the leg-openings 12. Of this height P, a length Q along which the slit 24 extends is dimensioned to be in a range of 1/4 to 3/4 of the height P.

Fig. 3 is a partially cutaway flatly developed plan view of the pants-type diaper of Fig. 1 wherein the front and rear waist regions 7, 8 have been separated from each other along the joint zones 9 and the respective halves of the functional sheet 20 have been separated from each other in the joint zone 23 so that the diaper 1 as a whole has been developed in the front-to-rear direction A as well as in the transverse direction B under tension. It should be noted here that, in Fig. 3, the diaper have been developed in this manner is designated with reference numeral 1a and the sheet before it is formed with the slit 24 is designated with reference numeral 20d.

Referring again to Fig. 3, the chassis 10 is hourglass-shaped and the peripheral edges 13 of the respective leg-openings 12 appear as the opposite side edges 13 of the crotch region 6. The respective side edges 13 convexly bow toward the front-to-rear center line F-F bisecting the width of the crotch region 6 in the developed diaper 1a. The respective pairs of the opposite side edges 7a, 8a of the front and rear waist regions 7, 8 are contiguous to the associated side edges 13 but extend in parallel to the front-to-rear center line F-F. The core 4 has a substantially rectangular shape and comprises a mixture 4a of fluff pulp and super-absorbent polymer particles wrapped with tissue paper 4b.

The functional sheet 20d shown by Fig. 3 lies on the inward side of the topsheet 2 so as to extend across the crotch region 6 and bonded by means of hot melt adhesive 49 extending also across the crotch region 6 to the topsheet 2 in the middle of the developed diaper 1a as viewed in the front-to-rear direction A, in other words, on or in the vicinity of the transverse center line G-G. The functional sheet 20d is bonded also to the topsheet 2 along the opposite side edges 13 of the crotch region 6 by means of hot melt adhesive 51. The functional sheet 20d has a front edge 51 and a rear edge 52 both extending in parallel to the transverse center line G-G and a distance between these front and rear edges 51, 52 is twice the height P in Fig. 2 and bisected by the transverse center line G-G. The functional sheet 20d further has front and rear zones 53, 54 to be joined respectively extending from the front and rear edges 51, 52 toward the transverse center line G-G along the front-to-rear center line F-F. Between these front and rear zones 53, 54 to be joined, a joint-free zone 57 is defined. The functional sheet 20d further has zones 55, 56 to be cut extending from the upper and lower edges 51, 52 toward the transverse center line G-G so as to partially bisect the functional sheet 20d in the transverse direction B and thereby to form the slit 24 in Fig. 1. The front and rear zones 53, 54 to be joined are cut along the front and rear zones 55, 56 to be cut in two before or after the functional sheet 20d and the topsheet 2 are or have been joined together in those front and rear zones 53, 54 to be joined. Along the front and rear edges 51, 52, the functional sheet 20d are folded back to form sleeves 61, 62 adapted to contain elastic members 63, 64 extending across the crotch region 6. The elastic members 63, 64 are bonded under tension but in a contractible manner to the respective inner surfaces of the sleeves 61, 62 by means of adhesive (not shown) applied intermittently along the longitudinal direction of these sleeves 61, 62 so that these elastic members 63, 64 may contract and cut off one from another as the functional sheet 20d is cut along the zones 55, 56 to be cut. Thus the elastic members 63, 64 are divided into the elastic members 41a, 41b, 42a, 42b of Fig. 1.

Without departing from the scope of the present invention, the functional sheet 20d in the illustrated embodiment itself being not elastically stretchable in the transverse direction may be replaced by a sheet made of material being elastically stretchable. In this case, the elastic members 63, 64 may be eliminated and the sheet may be bonded under or not under tension in the transverse direction B.

To obtain the pants-type diaper 1 of Fig. 1 from the developed diaper 1a, the crotch region 6 of the developed diaper 1a is folded back along the transverse center line G-G so that the front and rear waist regions 7, 8 may be put flat together with the functional sheet 20d inside. Then the front and rear waist regions 7, 8 are joined to each other in the joint zones 9 arranged along the respective pairs of the opposite side edges 7a, 8a thereof. As for the functional sheet 20d, the front zone 53 to be joined and the rear zone 54 to be joined are placed upon each other so that the zones 55, 56 to be may fall in a line with each other and then the zones 55, 56 to be cut may be cut to form the slit 24. The zones 53, 54 to be joined define the first joint zone 23a and the second joint zone 23b define the first joint zone 23a and the second joint zone 23b in Fig. 1 and the joint-free zone 57 defines a portion of the partition wall 35.

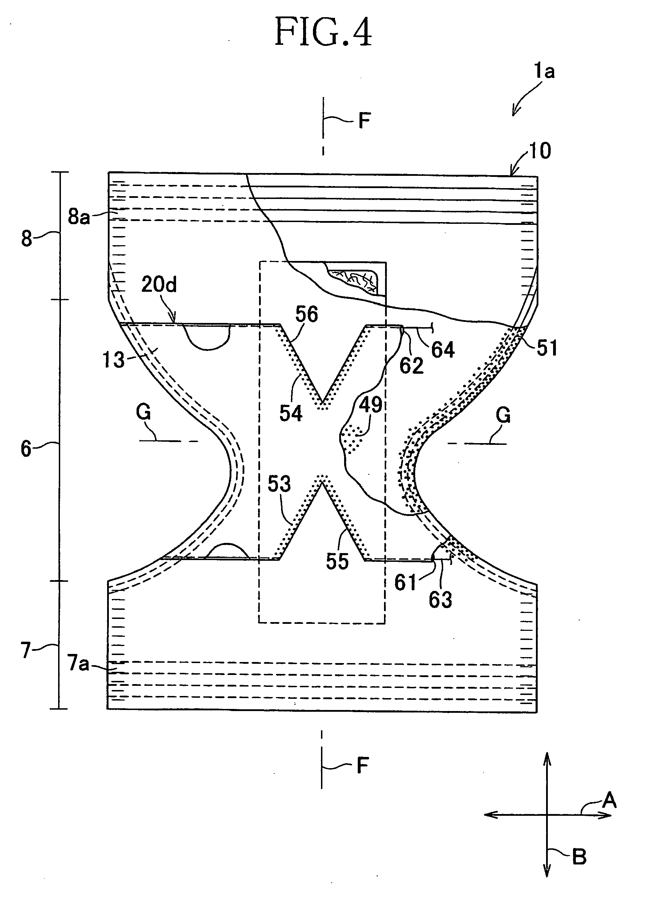

In the developed diaper 1a of Fig. 3, the hot melt adhesive 49 by means of which the functional sheet 20 is bonded to the topsheet 2 on the transverse center line G-G may extend only along a part of the width of the crotch region 6 (See Fig. 4) instead of extending over the entire width of the crotch region 6. However, the hot melt adhesive 49 preferably extends over the entire width as seen in Fig. 3 in view of a preventive effect against order that it is ensured to prevent urine and feces from being mixed together. Referring to Fig. 1, the slit 24 terminating short of the joint zone 23, i.e., not extending into the partition wall 35 is effective to prevent urine and/or feces from moving on the inner surface of the functional sheet 20 toward the leg-openings 12. In consequence, apprehensive contamination of the wearer's legs with urine and/or feces can be substantially reduced and leak-proof property of the diaper 1 can be correspondingly improved. When the requirement for the effect to prevent urine and feces from being mixed together is moderate, the present invention may be implemented without use of the hot melt adhesive 49.

Fig. 4 is a view similar to Fig. 3, showing another preferred embodiment of the invention. In the developed diaper 1a shown by Fig. 4, the joint zone in which the functional sheet 20d and the inner surface of the chassis 10 are bonded together along the transverse center line G-G is present in the middle of the crotch region 6 as viewed in the transverse direction B and not found in the vicinity of the opposite side edges 13 of the crotch region 6. The functional sheet 20d of Fig. 4 further includes front and rear V- or U-shaped zones 55, 56 to be cut replacing the slit 24 in the diaper 1 shown by Fig. 1. The front and rear zones 55, 56 to be cut respectively include the front zone 55 to be joined and the rear zone 54 to be joined. The front zone 55 to be cut and the rear zone 56 to be cut are symmetric with each other about the transverse center line G-G. In the pants-type diaper 1 wherein the front and rear zones 53, 54 to be joined are actually joined together so that the front and rear zones 55, 56 to be cut may fall in a line with each other, each of the leg-surrounding cuffs 20a, 20b defined by the functional sheet 20 at its both side edges has a dimension in the transverse direction B smaller than the leg-surrounding cuffs 20a, 20b in the embodiment shown by Fig. 1. By such dimensioning, the number of gathers (not shown) possibly formed in the leg-surrounding cuffs 20a, 20b can be correspondingly reduced. In the embodiment of Fig. 4 also, the functional sheet 20d made of material which is elastically stretchable in the transverse direction B may be used to eliminate the elastic members 63, 64.

## Claims

1. A disposable pants-type wearing article (1) having a front-to-rear direction, a transverse direction and a longitudinal direction being orthogonal one to another, and comprising a crotch region (6), a front waist region (7) extending forward from the crotch region and a rear waist region (8) extending rearward from the crotch region, said front and rear waist regions cooperating together to define a waist-opening (11) while said front and rear waist regions cooperate with said crotch region to define a pair of leg-openings (12), a peripheral edge of said waist-opening as well as respective peripheral edges (13) of said leg-openings being elastically stretchable and contractible in circumferential directions of the respective openings, and said wearing article further has a longitudinal center line extending in said longitudinal direction to bisect respective dimensions of said front and rear waist regions as measured in said transverse direction and a transverse center line extending in said transverse direction to bisect a dimension of said waist-opening as measured in said front-to-rear direction, said disposable pants-type wearing article being **characterized in that**:
said crotch region is provided on the inward side of the inner surface of said crotch region with a functional sheet (20) extending across said crotch region and bonded to said inner surface of said crotch region along opposite side edges thereof but left free from said inner surface of said crotch region between said opposite side edges, said functional sheet defines a partition wall (20c) bisecting said crotch region in said front-to-rear direction and a pair of leg-cuffs (20a, 20b) surrounding respective portions of the wearer's legs from inside thereof and thereby protecting said portions from contamination with bodily fluids; and
with said crotch region folded back along said transverse center line so that said front and rear waist regions may be put flat together, said functional sheet also is folded back along said transverse center line and in contact, on said transverse center line, with said inner surface of the crotch region, respective halves of said functional sheet folded back being formed in respective upper portions thereof with joint zones (23, 23a, 23b) on both sides of said longitudinal center line, said respective halves of said functional sheet folded back being joined together in said joint zones, said respective upper portions being bisected between said joint zones formed on the both sides of said longitudinal center line so that said respective upper portions bisected in this manner may define said leg-cuffs, respectively, and a portion of said functional sheet extending below said upper portions may be continuous in said transverse direction so as to define said partition wall; and wherein each of said upper portions of said functional sheet folded back onto itself occupies 1/4 to 3/4 of a dimension of said functional sheet having been folded back onto itself as measured in said longitudinal direction.

2. The wearing article according to Claim 1, wherein said functional sheet is joined to the inner surface of said crotch region on said transverse center line or in the vicinity thereof.

3. The wearing article according to Claim 1 or Claim 2, wherein said leg-cuffs are elastically stretchable and contractible in circumferential direction partially around the wearer's legs so that said leg-cuffs may elastically fit partially around said wearer's legs.

## Patentansprüche

1. Tragbarer Artikel von Einweghöschen (1), dessen Richtung von vorne nach hinten geht, eine Querrichtung und eine Längsrichtung, die rechtwinklig zueinander sind, und einen Schnittbereich (6), einen vorderen Taillenbereich (7), die sich vom Schnittbereich nach vorne erstreckt, und einen hinteren Taillenbereich (8), der sich vom Schnittbereich nach hinten erstreckt, umfassen, wobei die vorderen und hinteren Bereiche zusammenwirken, um eine Taillenöffnung (11) zu definieren, während die vorderen und hinteren Taillenbereiche mit dem Schnittbereich zusammenwirken, um ein Paar Beinöffnungen (12) zu definieren, wobei ein peripherer Rand der Taillenöffnung sowie die entsprechenden peripheren Ränder (13) in Umfangsrichtung der entsprechenden Öffnungen der Beinöffnungen elastisch dehnbar und zusammenziehbar sind, und der tragbare Artikel ferner eine längslaufende Mittellinie hat, die sich in Längsrichtung erstreckt, um die entsprechenden Dimensionen der vorderen und hinteren Taillenbereiche zu halbieren, wie in Querrichtung gemessen, und eine quer verlaufende Mittellinie, die sich in Querrichtung erstreckt, um eine Dimension der Taillenöffnung zu halbieren, wie in der Richtung von vorne nach hinten gemessen, wobei der tragbare Artikel von Einweghöschen **dadurch gekennzeichnet ist, dass**
der Schnittbereich auf der Innenseite der Innenfläche des Schnittbereichs mit einer funktionalen Bahn (20) ausgestattet ist, die sich quer über den Schnittbereich erstreckt und an der Innenfläche des Schnittbereichs an gegenüberliegenden Seitenrändern davon erstreckt, aber von den Innenflächen des Schnittbereichs zwischen den gegenüberliegenden Seitenrändern frei gelassen wird, wobei die funktionelle Bahn eine Trennwand (20c) definiert, die den Schnittbereich in Richtung von vorne nach hinten halbiert, und ein Paar Beinbündchen (20a, 20b), die die entsprechenden Abschnitte der Beine des Trägers von innen umgeben und somit die Abschnitte vor Verschmutzung mit Körperflüssigkeiten schützen, und
wobei der Schnittbereich an der quer laufenden Mittellinie zurückgefaltet ist, sodass der vordere und hintere Taillenbereich flach zusammengelegt wird, wobei die funktionale Bahn ebenfalls an der quer laufenden Mittellinie zurückgefaltet wird und an der quer laufenden Mittellinie mit den Innenflächen des Schnittbereichs in Kontakt ist, wobei die zurückgefalteten entsprechenden Hälften der funktionalen Bahn in den entsprechenden oberen Abschnitten davon mit Verbindungszonen (23, 23a, 23b) an beiden Seiten der längslaufenden Mittellinie geformt werden, wobei die entsprechenden Hälften der funktonalen Bahn zurückgefaltet und in den Verbindungszonen miteinander verbunden sind, wobei die entsprechenden oberen Abschnitte zwischen den Verbindungszonen halbiert werden, die auf beiden Seiten der längslaufenden Mittellinie gebildet werden, sodass die entsprechenden oberen Abschnitte auf diese Weise halbiert werden und die jeweiligen Beinbündchen definieren, und ein Abschnitt der funktionalen Bahn, die sich nach unten unter den oberen Abschnitten erstreckt, können in Querrichtung kontinuierlich sein, sodass sie die Trennwand definieren, und wobei jeder der oberen Abschnitte der funktionalen Bahn auf sich selbst zurückgefaltet ist und von ¼ bis ¾ einer Dimension der funktionalen Bahn einnimmt, die auf sich selbst zurückgefaltet ist, wie in Längsrichtung gemessen.

2. Tragbarer Artikel nach Anspruch 1, wobei die funktionale Bahn an der Innenfläche des Schnittbereichs an der querliegenden Mittellinie oder in der Nähe davon verbunden ist.

3. Tragbarer Artikel nach Anspruch 1 oder Anspruch 2, wobei die Beinbündchen in Umfangsrichtung teilweise um die Beine des Trägers elastisch dehnbar und zusammenziehbar sind, sodass die Beinbündchen teilweise elastisch um die Beine des Trägers passen.

## Revendications

1. Article d'habillement jetable de type culotte (1) ayant une direction allant de l'avant vers l'arrière, une direction allant dans le sens transversal et une direction allant dans le sens longitudinal de manière perpendiculaire les unes par rapport aux autres, et comportant une région au niveau de l'entrejambe (6), une région avant au niveau de la taille (7) s'étendant vers l'avant depuis la région au niveau de l'entrejambe et une région arrière au niveau de la taille (8) s'étendant vers l'arrière depuis la région au niveau de l'entrejambe, lesdites régions avant et arrière au niveau de la taille coopérant ensemble pour définir une ouverture pour la taille (11) alors que lesdites régions avant et arrière au niveau de la taille coopèrent avec ladite région au niveau de l'entrejambe pour définir une paire d'ouvertures pour les jambes (12), un bord périphérique de ladite ouverture pour la taille ainsi que des bords périphériques respectifs (13) desdites ouvertures pour les jambes étant en mesure de s'étendre et de se contracter de manière élastique dans des directions allant dans le sens de la circonférence des ouvertures respectives, et ledit article d'habillement ayant par ailleurs une ligne centrale longitudinale s'étendant dans ladite direction allant dans le sens longitudinal pour diviser des dimensions respectives desdites régions avant et arrière au niveau de la taille telles qu'elles sont mesurées dans ladite direction allant dans le sens transversal et une ligne centrale transversale s'étendant dans ladite direction allant dans le sens transversal pour diviser une dimension de ladite ouverture pour la taille telle qu'elle est mesurée dans la direction allant de l'avant vers l'arrière, ledit article d'habillement jetable de type culotte étant **caractérisé en ce que** :
ladite région au niveau de l'entrejambe comporte sur le côté intérieur de la surface intérieure de ladite région au niveau de l'entrejambe une feuille fonctionnelle (20) s'étendant en travers de ladite région au niveau de l'entrejambe et contrecollée sur ladite surface intérieure de ladite région au niveau de l'entrejambe le long des bords latéraux opposés de celle-ci mais laissée libre par rapport à ladite surface intérieure de ladite région au niveau de l'entrejambe entre lesdits bords latéraux opposés, ladite feuille fonctionnelle définissant une paroi de séparation (20c) divisant ladite région au niveau de l'entrejambe dans ladite direction allant de l'avant vers l'arrière et une paire de revers au niveau des jambes (20a, 20b) entourant des parties respectives des jambes de l'utilisateur depuis l'intérieur de celles-ci et pour ainsi protéger lesdites parties contre toute contamination par des fluides corporels ; et
ladite région au niveau de l'entrejambe étant repliée le long de ladite ligne centrale transversale de telle sorte que lesdites régions avant et arrière au niveau de la taille peuvent être assemblées à plat ensemble, ladite feuille fonctionnelle est aussi repliée le long de ladite ligne centrale transversale et en contact, sur ladite ligne centrale transversale, avec ladite surface intérieure de la région au niveau de l'entrejambe, les moitiés respectives de ladite feuille fonctionnelle repliée étant formées dans des parties supérieures respectives de celle-ci avec des zones de jointure (23, 23a, 23b) des deux côtés de ladite ligne centrale respective, lesdites moitiés respectives de ladite feuille fonctionnelle repliée étant jointes ensemble dans lesdites zones de jointure, lesdites parties supérieures respectives étant divisées entre lesdites zones de jointure formées des deux côtés de ladite ligne centrale longitudinale de telle sorte que lesdites parties supérieures respectives divisées de cette manière peuvent définir lesdits revers au niveau des jambes, respectivement, et une partie de ladite feuille fonctionnelle s'étendant sous lesdites parties supérieures peut être continue dans ladite direction allant dans le sens transversal de manière à définir ladite paroi de séparation ; et dans lequel chacune desdites parties supérieures de ladite feuille fonctionnelle repliée sur elle-même occupe 1/4 à 3/4 d'une dimension de ladite feuille fonctionnelle ayant été repliée sur elle-même tel qu'il a été mesuré dans ladite direction allant dans le sens longitudinal.

2. Article d'habillement selon la revendication 1, dans lequel ladite feuille fonctionnelle est jointe sur la surface intérieure de ladite région au niveau de l'entrejambe sur ladite ligne centrale transversale ou à proximité de celle-ci.

3. Article d'habillement selon la revendication 1 ou la revendication 2, dans lequel lesdits revers au niveau des jambes sont en mesure de s'étendre et de se contracter de manière élastique dans une direction allant dans le sens de la circonférence partiellement autour des jambes de l'utilisateur de telle sorte que lesdits revers au niveau des jambes peuvent s'adapter de manière élastique partiellement autour desdites jambes de l'utilisateur.
